# EUROPEAN PATENT APPLICATION

(11) **EP 1 417 932 A2**
(43) Date of publication of application: **12.05.2004**
(21) Application number: 03425635.4
(22) Date of filing: 30.09.2003
(51) Int. Cl.: A61B 10/00

(54) **Device for needle-biopsy**

(30) Priority: 07.11.2002 IT fi20020216
(71) Applicant: Gironi, Aurelio, 59100 Prato (IT)
(72) Inventor: Gironi, Aurelio, 59100 Prato (IT)
(74) Representative: Martini, Lazzaro

(57) **Abstract**

A device for carrying out drawings by means of bioptic needles consisting of means for drawing fragments or organic samples on diagnostic purposes, guide and support means for said drawing means, means for controlling the forward motion of said drawing means, characterised in that it consists of damping means for the forward motion of said drawing means (Fig.1).

## Description

The present invention relates to a device for needle-biopsy.

It is well known that drawings by means of bioptic needles imply the use of needle cannulae which hold tissue samples or organs drawn from human beings on diagnostic purposes.

These needles basically consist of a sharp and hollow cannula and of a stylet placed inside the cannula, so that it can slide through it, which functions as an occlusion core, to prevent foreign material from penetrating into the needle during the positioning phase of the needle cannula in the area where the drawing is to be carried out.

The well known devices commonly used for carrying out drawings by means of bioptic needles, in addition to a group cannula/stylet, comprise means for the separate motion of the cannula and of the stylet. The well known devices can be divided into three categories:
- manual devices, in which both the motion of the stylet and the motion of the cannula are manually controlled by the operator;
- semiautomatic devices, in which the exit of the stylet is manually operated, while the motion of the cannula is determined by the action of a spring activated by the operator who acts on a pushbutton of the device;
- automatic devices, in which both the stylet and the cannula are moved by means of to corresponding springs released by the operator who, as in the above case, acts on a button foreseen on the body of the device.

The well known devices cannot be used whenever a gradual forward motion rather than an impulsive forward motion of the stylet is required. In these cases it is necessary to use semiautomatic devices.

Therefore it is evident that the operator needs to have several drawing devices and to choose them according to the type of operation he is required to carry out.

The present invention basically aims at eliminating this inconvenience.

These results have been achieved in compliance with the invention thanks to the idea of realising a device having the features described in the independent claims. Further features are the subject of the dependent claims.

Thanks to the present invention, it is possible to use the same device both for operations requiring a gradual and manual forward motion of the stylet and of the cannula and for operations allowing the impulsive operation of the stylet and of the cannula. Moreover it is possible to obtain a slow forward motion of the stylet and of the cannula, at a speed which can be adjusted by the operator, even in automatic mode operation of the device, so as to reduce the traumatic effects of the operation on the adjacent tissues. Moreover, thanks to a particular structure of the operating system of the device in automatic operation, its use is extremely comfortable and safe. The device in compliance with the invention offers further advantages as, thanks to it, the assembly of the needle cannula and the recharge of the device after it has been used can be easily carried out.

The technicians who work in this field can better understand further advantages and features of the present invention thanks to the following description and to the enclosed drawings, as a practical exemplification of the invention, which are not to be considered in a restrictive sense, in which:
- Fig. 1 shows a longitudinal section view of the device according to the invention;
- Fig.2 shows a longitudinal section view of the device according to a section plane orthogonal to that of Fig.1;
- Fig. 3 shows the device of Fig.1 without cannula and stylet;
- Fig. 4A shows a longitudinal section view of the group cannula-stylet;
- Fig.4B shows a longitudinal section view of the group cannula-stylet according to a section plane orthogonal to that of Fig.4A;
- Fig.5 shows a longitudinal section view of the control unit for the forward motion of the stylet;
- Figs 6A-6D respectively show a plant view of bushing 21, a lateral view of this bushing, a section view according to A-A and a section view according to B-B of Fig.6A;
- Fig.7 shows a longitudinal section view of the unit formed by casing 19 and by bushing 21;
- Figs 8A and 8B respectively show a view of the device with rod 13 in a free position and of rod 13 which is held, according to "X" of Fig.1;
- Figs 9A-9D respectively show a view of body 23 according to "X" of Fig.1, a section view according to C-C of Fig.9A, a view according to "Y" of Fig.9B and a section view according to D-D of Fig.9C;
- Figs 10A-10E respectively show a plant view of safety device 38, a section view according to E-E of Fig.10A, a section view according to F-F of Fig.10A, a lateral view of the device showed in Fig.10A and a section view according to G-G of Fig.10D;
- Fig.11-A shows a plant view of adjusting bearing 12;
- Fig. 11B is a section view according to H-H of Fig. 11A;
- Fig. 12 shows a plant view of adjusting bearing 11;
- Fig.13 shows a lateral view of the adjusting bearing showed in Fig.12 on rod 10;
- Figs 14A-14C show three possible relative positions of adjusting bearings 11 and 12;
- Figs 15A-15C respectively show a plant view of casing 19, a section view according to K-K and a section view according to L-L of Fig.15A;
- Fig.16A shows a plant view of the group formed by elements 19 and 21;
- Fig. 16B shows a section view according to M-M of Fig.16A;
- Fig. 16C shows a view which is analogous to that of Fig. 16A, but with element 21 rotated of 90°;
- Fig. 16D shows a section view according to N-N of Fig.16C;
- Fig. 17A shows a device in compliance with the invention in automatic operation without damping during the release phase of the cannula support;
- Fig. 17B shows the device of Fig.17A at the end of the forward motion of the cannula support;
- Figs 18A and 18B r correspond to Figs 17A and 17B respectively, as regards the automatic operation mode of the device with damping;
- Figs 19A and 19B correspond to Figs 17A and 17B as regards the semiautomatic operation mode of the device;
- Fig. 20A shows a longitudinal section view of a device in compliance with a further embodiment of the present invention;
- Fig. 20B shows a view of the device of Fig.20A equipped with a drawing group in which only the cannula moves forward;
- Fig. 20C shows a view of the device of Fig. 20A, equipped with a drawing group which foresees first the forward motion of the stylet and then that of the cannula;
- Fig. 21A shows a lateral view of the needle cannula group mounted on the device of Fig. 20B;
- Fig. 21B shows a section view according to G-G of Fig.21A;
- Fig. 21C shows an enlarged detail of Fig. 21A;
- Fig. 22A shows a lateral view of the needle cannula group mounted on the device of Fig.20C;
- Fig. 22B shows a section view according to Q-Q of Fig.22A;
- Fig.22C shows an enlarged detail of Fig.22B;
- Fig. 23A shows body 3 of Fig.20A seen from body 4;
- Fig.23B shows a section view according to Y-Y of Fig.23A;
- Fig.24A shows body 8 of Fig.20A seen from body 3;
- Fig.24B shows a section view according to W-W of Fig.24A;
- Fig.25A represents body 4 of fig. 20A seen from the side opposite to that of body 3;
- Fig.25B shows a section view according to Z-Z of Fig.25A.

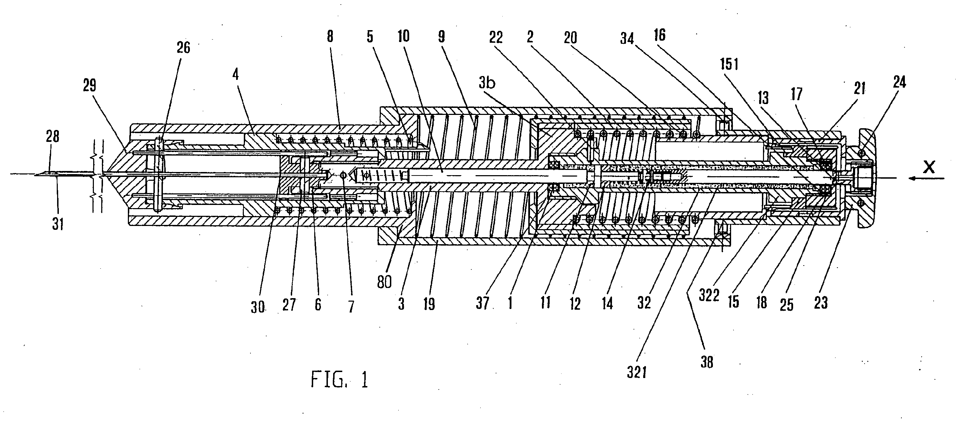

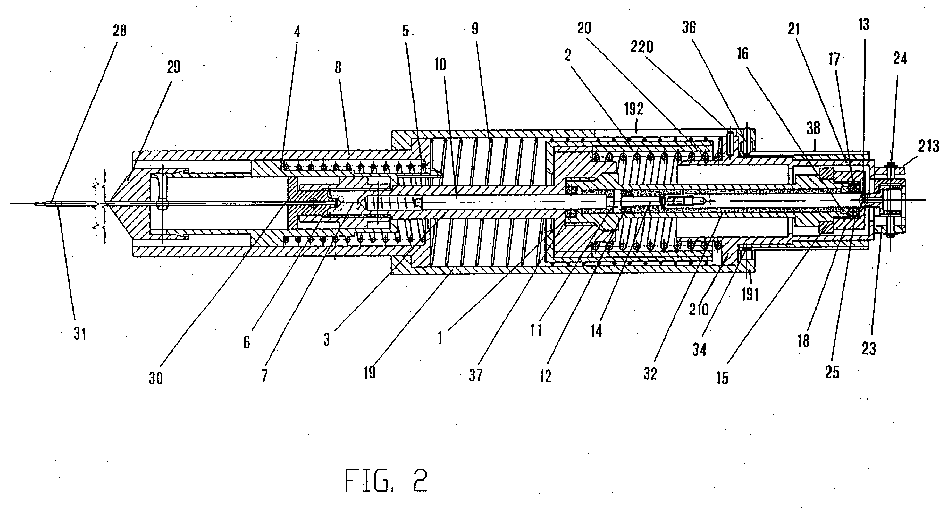

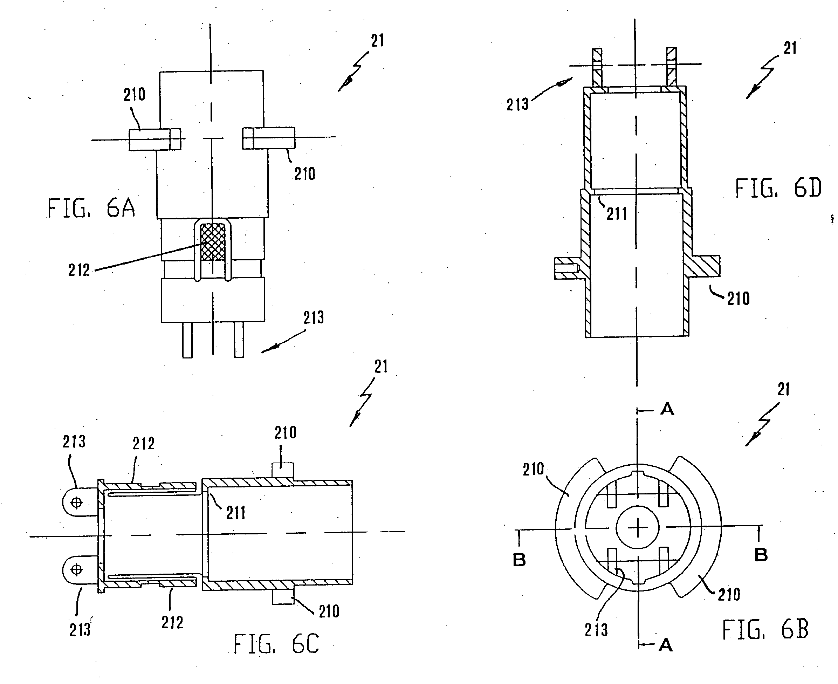

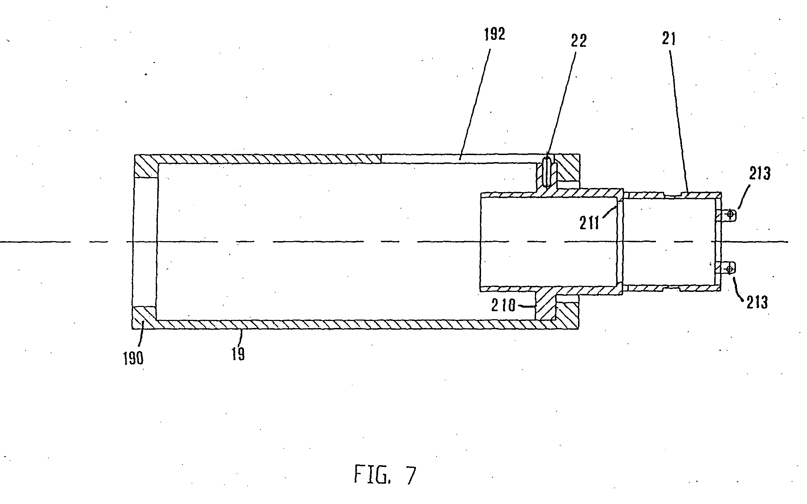

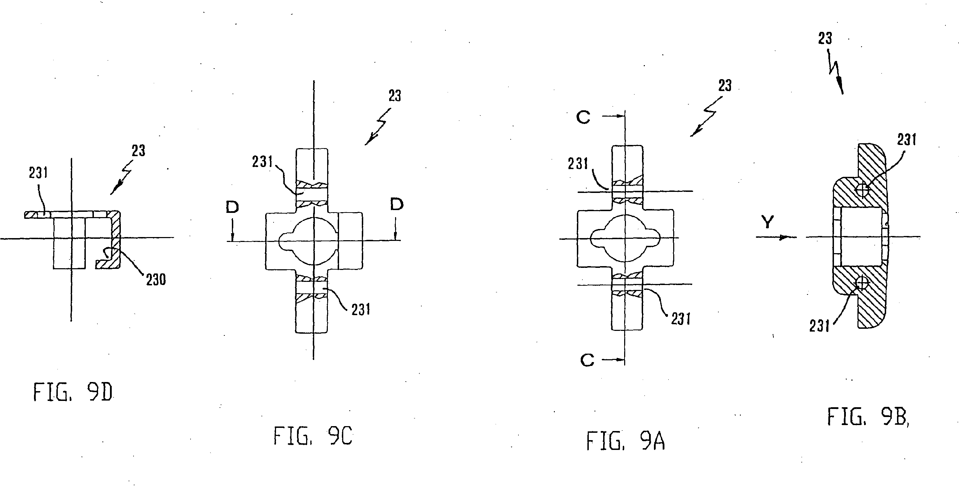

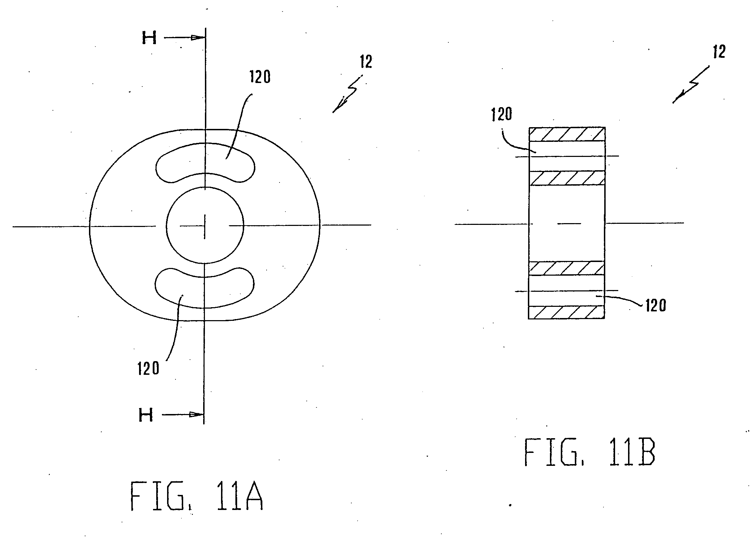

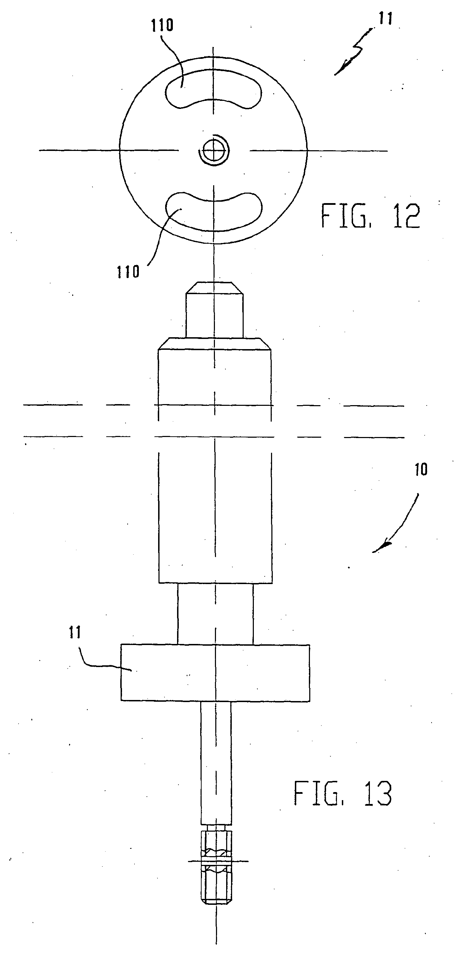

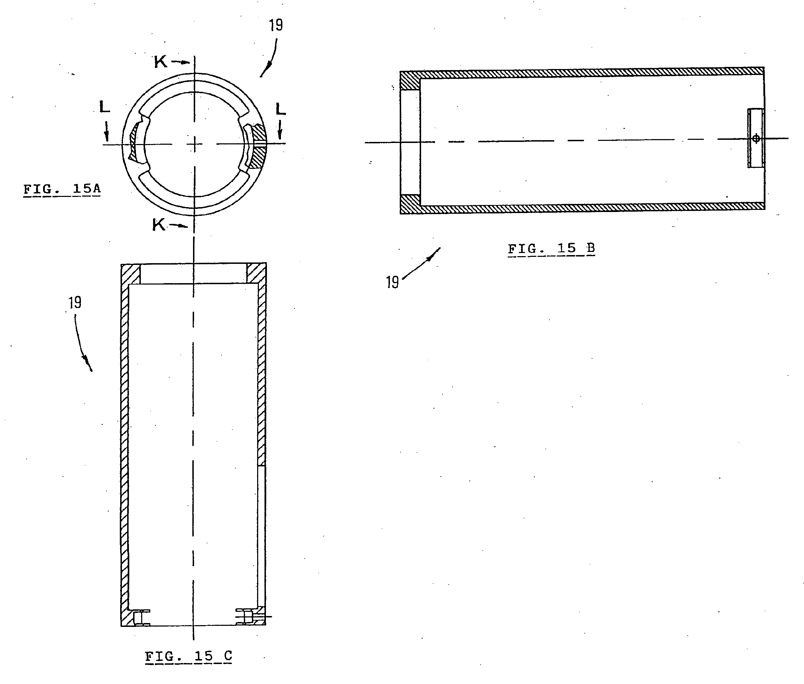

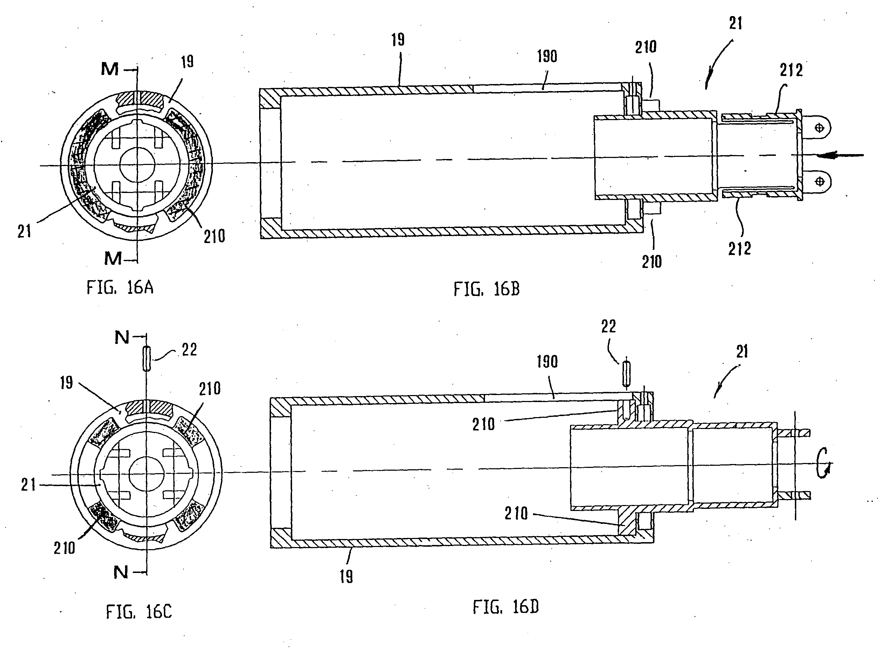

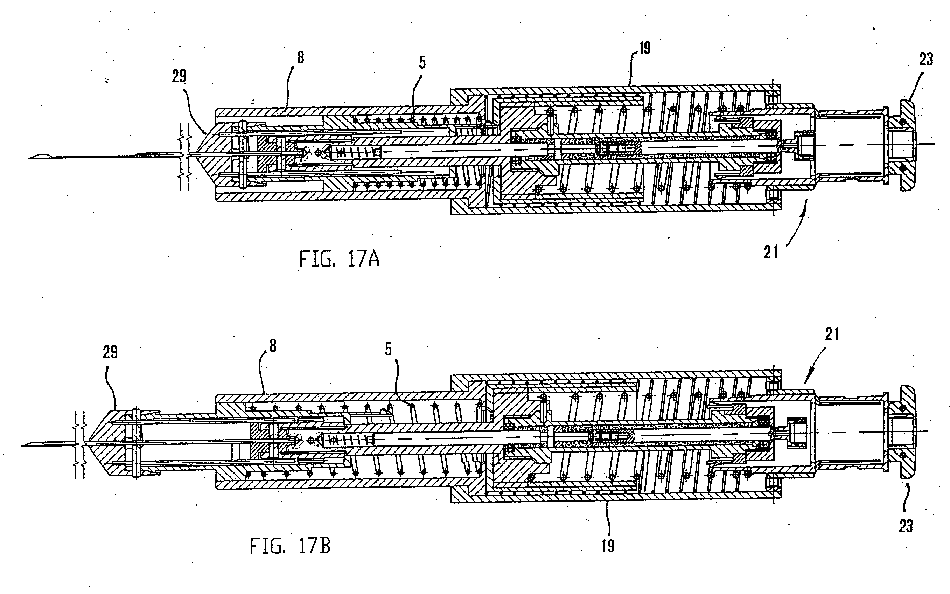

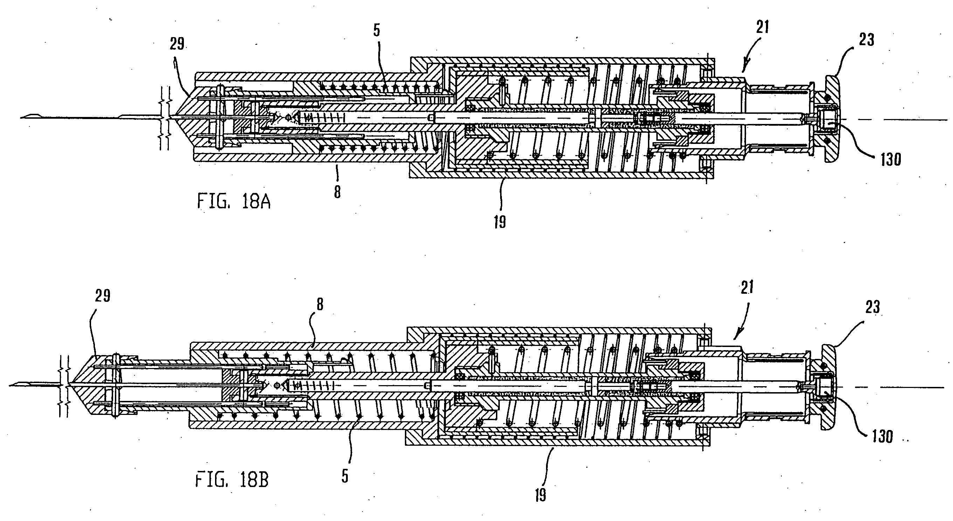

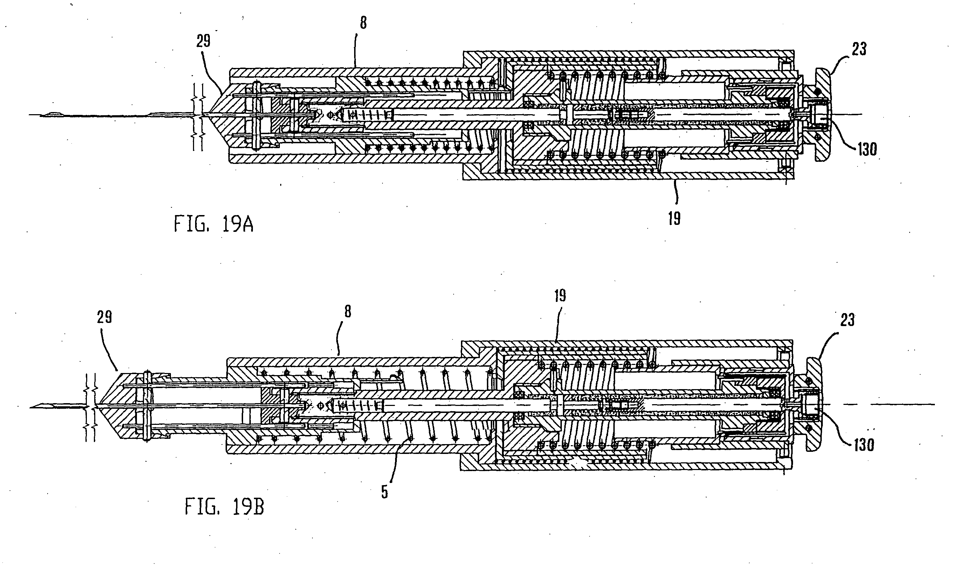

With reference to Figs 1-19B of the enclosed drawings, stylet (28) and cannula (31) are fixed to the corresponding support groups or units (6, 4); stylet (28) is fixed to group (6) and cannula is fixed to group (4). In turn, supports (6, 4) of the stylet and of the cannula are associated with the corresponding control unit or organ (20, 5): the organ (20) determines the forward motion of support (4), as better described in the following. the stylet (28) is mounted inside the cannula so as to slide through it (31).

The support (4) consists of a tubular body with a radial web (40) featuring three angularly equidistant elastic appendages (400) at its back base. The free end of each appendage (400) is hook shaped. A cap (29) featuring a hole having a longitudinal central channel, through which the stylet (28) is positioned so as to pass through it, that is to say so as to slide through it, is inserted on the other base of the support (4): being the cannula (31) axially fixed to said cap (29).

The cap (29) is fixed to the body (4) by means of a section pin (26) which features a central hole through which the stylet (28) passes. The support (6) is placed inside the support (4) and features a basically tubular structure with an external radial web (60) which fits with the internal wall of support (4). The body (6) is threaded in its interior, so that it can be screwed on the distal end of axial peg (3). A pin (7) is transversely placed so that it can pass through the body (6) and the peg (3) to allow its assembly. The holes foreseen in body (6) and in peg (3) for the assembly of pin (7) allow this assembly only if the angular position of body (6) in relation to body (3) corresponds to a well known and predetermined angular position, as is better described in the following. The stylet (28) is fixed to the body (6) by means of a distal fixing body (30) which is attached to the body (6) by means of a section pin (27) the ends of which jut out of the profile of body (6) without interfering with the internal wall of body (4). Two thin rods (293) orthogonally passing through two corresponding holes of body (30) connect cap (29) with said fixing body (30).

Said bodies (4) and (6) are placed inside a tubular sleeve (8) so that the ring web (40) of the body (4) is in contact with the internal wall of the sleeve. The back base (80) of the latter is flange shaped and positioned so as to be in contact with the front base (190) of a cylindrical casing (19), said base (190) features a central opening for the positioning of the sleeve (8) which is coaxial to the casing itself (19). The sleeve (8) juts out of the front base (190) of casing (19). A spring (5) is mounted between web (40) of body (4) and flange (80) of sleeve (8) which is coaxial and external to body (4) but internal to sleeve (8). The hook shaped ends of appendages (400) of body (4) are fixed to the surface of flange (80) when the device is ready for use.

Said peg (3) features a cavity which features a longitudinal opening and ends with a bucket (3b) on the side opposite to the fixing side of body (6) in which the head (320) of a tubular body (32), which is coaxial to and in contrast with peg (3), is screwed. An interconnection pin (22) is placed between bucket (3b) and peg (3) of body (32) A rod, which passes through bucket (3b) and through the threaded head of body (32. A rod (10) which passes through the bucket (3b) and through the threaded head (320) of the body (32) is inserted in the above longitudinal cavity of peg (3) and is connected with a coaxial pin (13) on the opposite side. The connection between rod (10) and pin (13) takes place by means of a spring cotter (14).

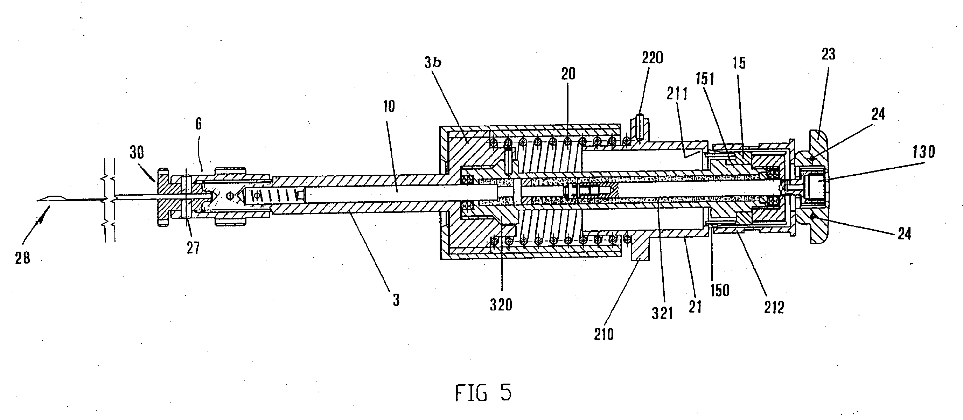

Fig. 1 and Fig.5 show an annular gasket (37) placed on the rod (10) and a flat gasket (1) between the bucket (3b) and the head (320) of the body (32) through whose opening the rod (10) passes. On the opposite side, an analogous gasket (17) is foreseen between a ring (16) mounted on the body (32) and a plug (18) is screwed on the head portion of the body (32).

The pin (13), the threaded portion of the rod (10) and the bearings (11) and (12) are in the cavity of the tubular body (32), which cavity contains a predetermined amount of oil or a similar compressible or incompressible fluid (321). In practice, the tubular body (32) functions as a tank for the fluid (321) and a casing for the threaded portion of rod (10), for the pin (13) and for the bearings (11) and (12).

A bearing (11) is present on the rod (10) with two diametrically opposed eyelets, said bearing (11) being joined to the rod (10), so each rotation of the latter around its longitudinal axis causes a corresponding rotation of bearing (11).

Another bearing (12) facing the above bearing (11) is positioned on the rod (10) with no possibility of rotation. Also the bearing (12) is provided with eyelets (120).

The bearings (11) and (12) are placed inside the chamber bounded by the above body (32) so that they subdivide this chamber into two sections, one upstream bearing (12) and the other downstream bearing (11).

The plug (18) features a central opening through which the back side of pin (13) projects. The latter ends with a knurled head (130) which allows the body to rotate both in the clockwise and in the anticlockwise direction, so as to adjust the reciprocal position of bearings (11) and (12), since the bearing (12) is in a fixed angular position in relation to the body (32). A cap shaped element (23) is mounted over the head of the pin (13). This element (23) features two transverse holes (231), through which pass two corresponding pins (24) fixed on reliefs (213) of bushing (21).

Said pins (24) function as guide elements for cap (23) which, therefore, can be moved according to the direction of pins (24).

Moreover, element (23) features a hook shaped internal body (230), so, when element (23) slides on pins (24), it determines the position of the cap (23) in which position the head (130) is hooked by the body (230). The functions of this constructive detail are described in the following.

The external surface of bucket (3b) is threaded and a cylindrical bearing (2) whose surface surrounds bucket (3) and extends inside casing (19) along a predetermined run length, is screwed on it.

A bushing (21) which is coaxial to and external to tubular chamber (32) is partially inserted in the cavity of bearing (2).

The bushing (21) features an annular projection (210) which is in contact with the internal back base (191) of casing (19). Moreover, a pin (220) is transversely positioned in said projection (210) and juts out of said ledge so as to be partially placed inside a longitudinal eyelet (192) of the casing (19).

Said pin (220) hinders the reciprocal rotation of bearing (21) and of casing (19) when the device is assembled.

The pins (220, 22, 7) indicate the univocal angular position of elements (4, 6) with which the cannula and the stylet are associated.

A spring (9), which is coaxial and external to the bushing (2) is positioned between the flange (80) of the sleeve (8) and the projection (210) of the bushing (21).

A spring (20), which is coaxial and internal to the bearing (2), is positioned between the bucket (3b) of the element (3) and the projection (210) of the bushing (21). By screwing or unscrewing bearing (2) on the thread of the bucket (3b) it is possible to adjust its distance from the appendages (400) of the body (4). The axial position of the bearing (2) can be seen through the above eyelet (192), that is to say it is possible to see the motion of the group cannula/stylet as described in the following.

The bushing (21) projects beyond the central light of casing (19), on the side turned towards the knurled head (130) of pin (13).

The bushing (21) features two centripetally flexible elements (212), that is to say two diametrically opposed buttons, whose free ends are turned towards seam (211) of bushing (21), on the side turned towards cap (23).

On its head, bushing (21) features two reliefs (213) with eyelets, in which the above pins (24) are positioned.

A ring (15) is associated with the bushing (21) and with the body (32). This ring features two flexible appendages (15) which are placed in the interior and are facing the buttons (212) of the bushing (21). The free end (which is turned towards the group cannula/stylet) of each appendage (150) rests on the edge of an internal seam (211) of the bearing (21).

The ring (15) is inserted on the body (32) and rests on the edge of a diametrical web (322) of this body. A tooth (151) of ring (15) is inserted in a corresponding cavity presented by said web (322) of the body (32). An annular gasket (25) rests on a ring (16) which is associated to said web (322) so as to be positioned between the pin (13) and the cap (18).

In practice, the body (32) and the bushing (21) are reciprocally joined by means of the appendages (150) presented by the ring (15) which, in this way, functions as interconnection element between the body (32) and the bushing (21).

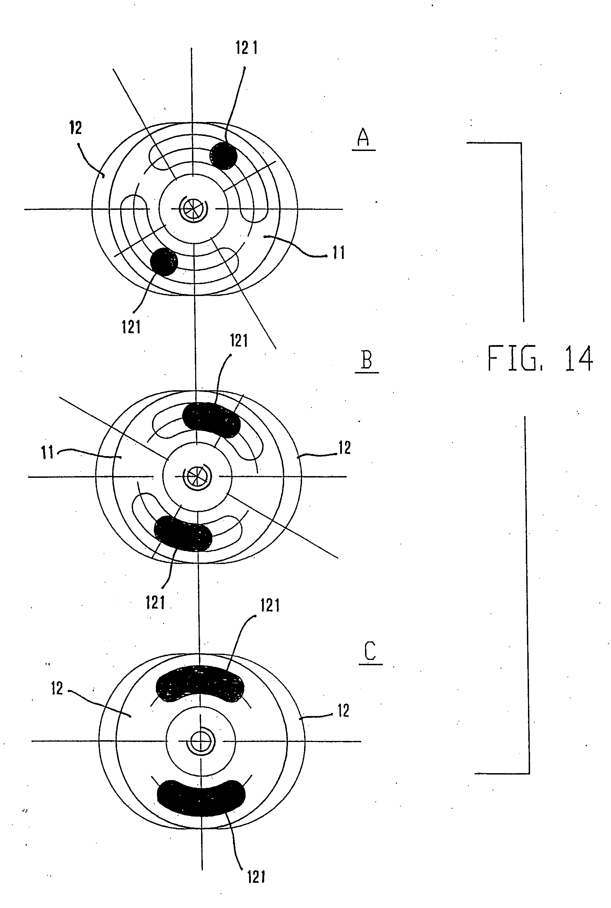

It is possible to adjust the relative position of the bearings (11) and (12) by rotating the rod (10), that is to say by manually acting on the head (13) of the rod (13) or, more exactly, by rotating the bearing (11) in relation to the bearing (12). In this way, it is possible to adjust the damping assured by the fluid (321). For example, in Fig. 14A, the relative position of vision slits (110, 120) presented by the bearings (11, 12) determines a minimum communication opening (121) between the two sections into which the chamber bounded by the body (32) is subdivided, which corresponds to maximum damping. In Fig.14B said opening (121) has and intermediate amplitude. In Fig.14C, the opening (121) is maximum and the damping effect assured by the fluid (321) is minimum.

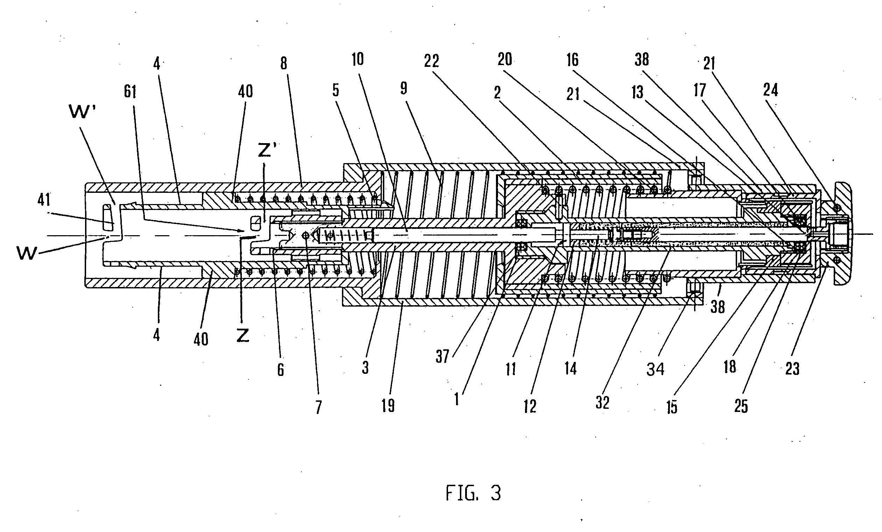

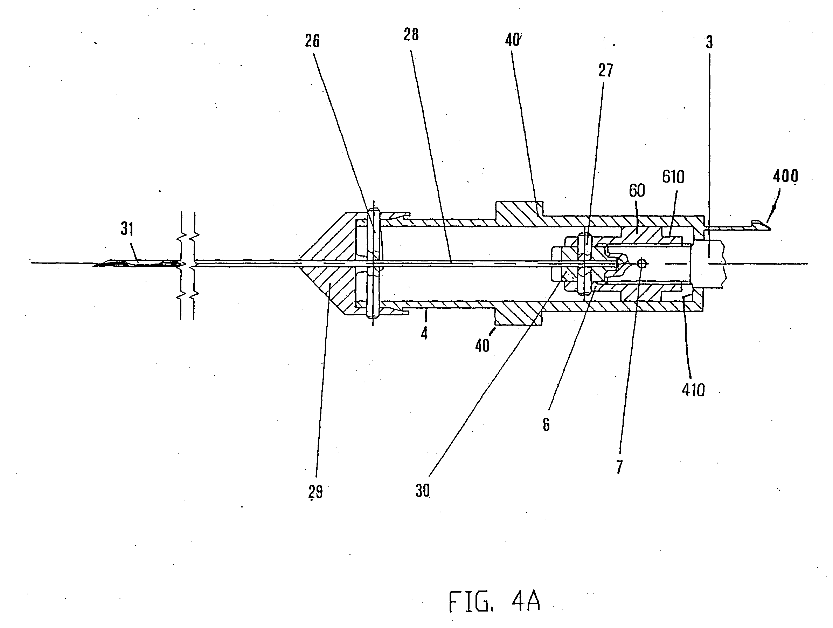

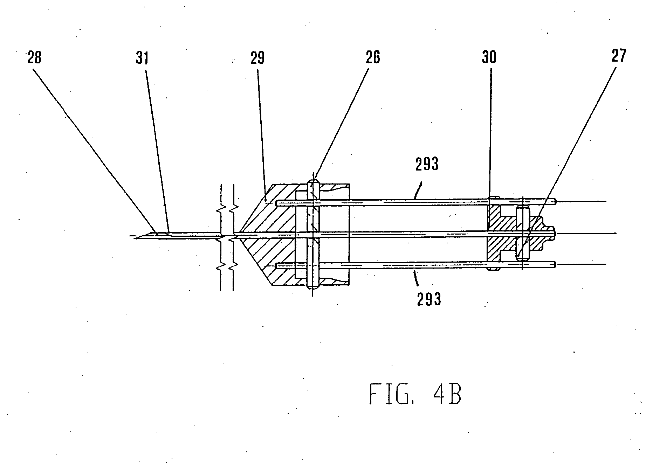

As regards the assembly of the group cannula/stylet the unit illustrated in Fig.4B is introduced into main body (4), so that the pin (26) is inserted in an L "shaped" hole (41) which is presented on the front by the body (4) so that the pin (27) is inserted in the corresponding hole (61) of the body (6). The positions which the pins (26, 27) respectively assume are indicated in this phase with (W) and (Z) as illustrated in Fig. 3. Said unit is subsequently rotated of 90° so as to allow the pin (27) to be introduced into the hole (41) with a slight elastic strain, while the pin (27) is placed in the final stretch of the hole (61). The final positions of the pins (26, 27) are indicated by (W') and (Z') in Fig.3.

A device in accordance with the invention can be provided with a safety device (38) for isolating the above flexible appendages (150) of the ring (15).

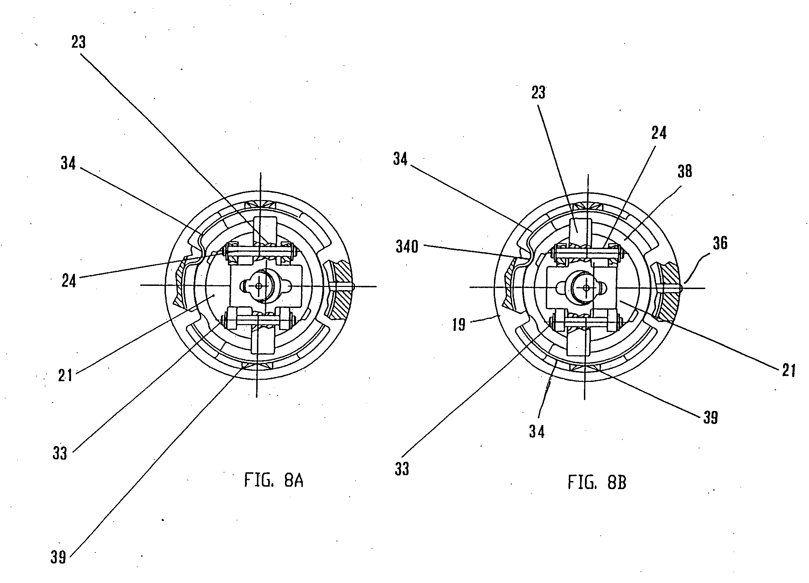

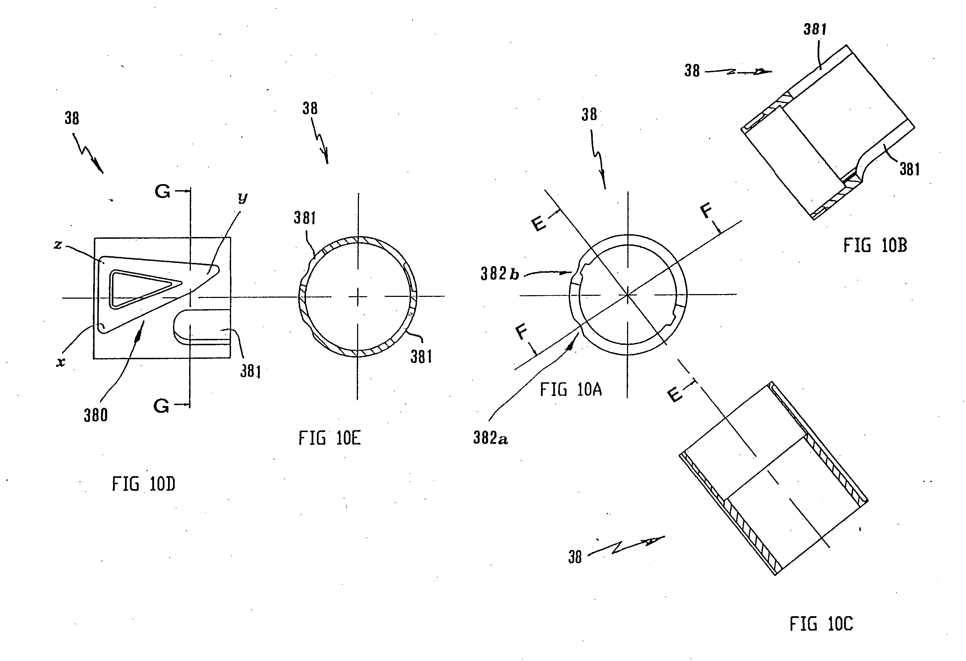

According to the example illustrated in Figs 10A-10E of the enclosed drawings, said device (38) consists of a tubular body whose external surface features a depressed track (380) resembling a right angled triangle with one cathetus parallel to the longitudinal axis of the body itself. The latter is coupled with the above bushing (21) and capable of rotating around its longitudinal axis as illustrated in Figs 1, 2 and 3. During the recharge phase of the drawing device, that is to say during the repositioning phase when the device is ready for use, casing (19) is held with one hand, while cap (23) is pressed with the palm of the other hand, so as to exert the pressure necessary to move the bearing (21) and the body (38) forward inside the casing (19). As a consequence of this, the pin (36) is in contact with the track (380) of the body (38), in the initial phase at the "X" point, and, due to the forward motion of the body (38) in relation to the pin (36), which is fixed, at the end of the recharge run the pin is at the "y" vertex of track (380). As the "xy" run of track (380) corresponds to the hypothenuse of the right angled triangle defined by the track and as said run intersects the longitudinal axis of the body (38) with the forward motion of latter, the pin (36) moves along track (380) and determines the rotation of body (38). The rotation is interrupted when the pin (36) is in the "x" point. In this position, the lateral surface of body (38) covers the buttons (212) of bushing (21) and renders them inaccessible. The body returns to the respective starting position by releasing bushing (21) due to the action of spring (9), while pin (36) slides through the "yz" run of the track (380). At the end of the return motion of bearing (21), pin (36) is in the "z" point. As the "yz" run of the track (380) is parallel to the longitudinal axis of body (38), during the return motion of bushing (21), there is no further rotation of the body (38). In this configuration, the device can be operated in semiautomatic mode. In order to activate the drawing device after said recharge, the operator can rotate the body (38) manually, so as to allow pin (36) to move along "zx" run of track (380), and to find itself in the "x" point, so as to allow the two longitudinal slits (381) of body (38) to be in contact with buttons (212) which become accessible again. Moreover, the external surface of body (38) features two undercuts (382a, 382b) which determine respective position in order to maintain the safety position or the non allowed automatic operation and the allowed automatic operation, in cooperation with a flexion spring provided with an internal appendage (340) which selectively comes in contact with said undercut (382a) or (382b). The spring (34) is showed in Fig.8A and in Fig.8B. These figures show some Seeger rings (33) for the pins (24) and some seats (39) presented by the casing (19) to keep the spring (34) in a coaxial position inside the casing (19), but external to the body (38).

The following description refers to the present device in automatic operation.

When the operator presses buttons (212) of the bushing (21), the elastic appendages (150) of the ring (15) are released from the bushing (21), that is to say from the seam (211), so as to allow the bushing (21) to disengage from body (32) and the spring (20) to act on the bucket (3b). As a consequence of this, the rod (3) advances and pushes the body (6) and the stylet (28) forward. This forward motion is interrupted when bushing (2) impacts on appendages (400) of the body (4). Due to the impact on the bushing (2), said appendages (400) centripetally bend and are unhooked from the flange (80) so as to allow spring (5) to push body (4) to move forward and, as a consequence of this, to allow cannula (31) to move forward. This forward motion is interrupted in the contact point between external back base (610) of the body (6) and internal back base (410) of the body (4).

If the operator has previously placed the cap (23) in such a way as to allow the head (13) of the pin (13) to be hooked, the action of the spring (20) is hindered by the strength exerted by the fluid (321) when it passes through the openings of the bearings (11, 12), whose reciprocal positions are determined by the operator by simply rotating the head (130) of the pin (13) as previously said, according to the damping degree of the desired action. When the cap (23) is hooked to the pin (13), the pin (13) remains fixed, while the tubular body (32) moves forward due to the action of the spring (20), but this forward motion is hindered by the passage of the fluid (321) through the openings of the bearings (11) and (12).

When the cap (23) is unhooked from pin (13), as in the above case, the latter can impulsively move forward with the stylet support unit (6).

The operator can operate the device in semiautomatic mode by exerting a manual pressure on the cap (23) without pressing the buttons (212), so as to allow the stylet (28) (in other words the relevant support unit (6)) to advance manually, until the back base of the bearing (2) comes in contact with the appendages (400) of the unit (4) which supports the cannula (31).

At this point, the impulsive forward motion of the unit (4) and of the cannula (31) takes place as described for the automatic mode operation of the device.

The interaction of the group stylet/cannula with the tissue sample to be drawn is identical to that of traditional devices, so this interaction is not described in detail.

After the device has been operated in automatic or semiautomatic mode, the operator can disassemble the device for the recovery of the sample by acting in the reverse order.

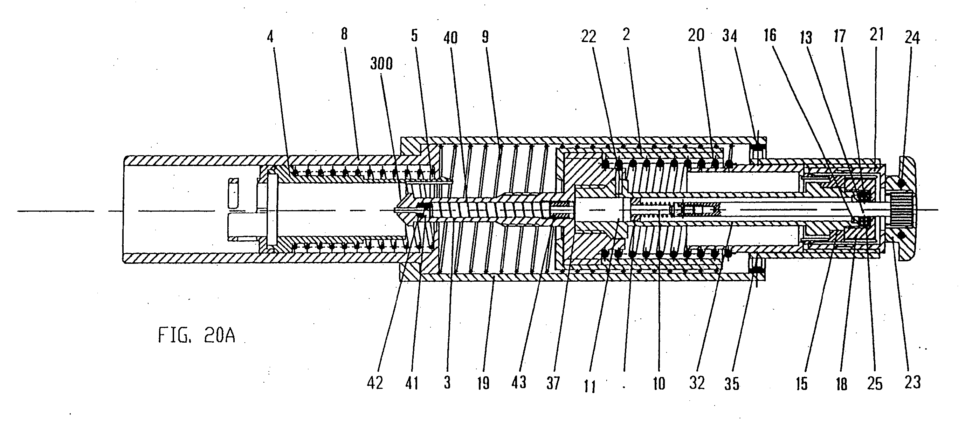

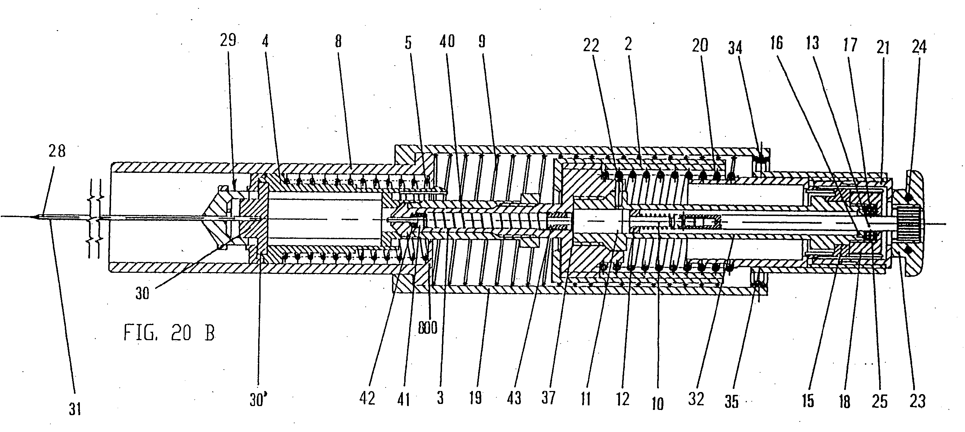

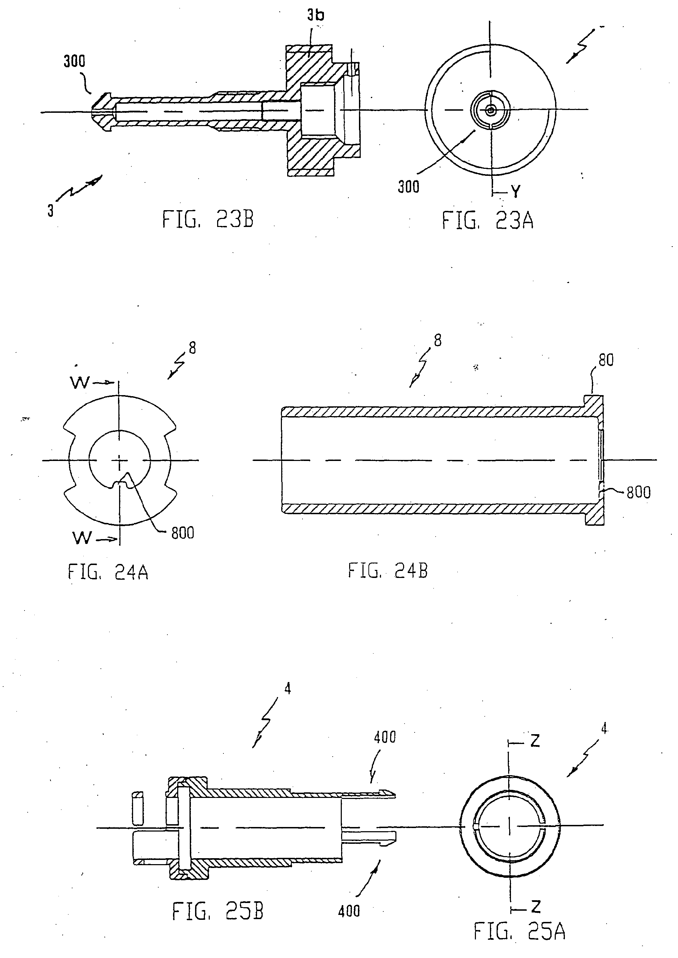

The examples illustrated in Figs 20A-25B of the enclosed drawings foresee a further embodiment of the present device.

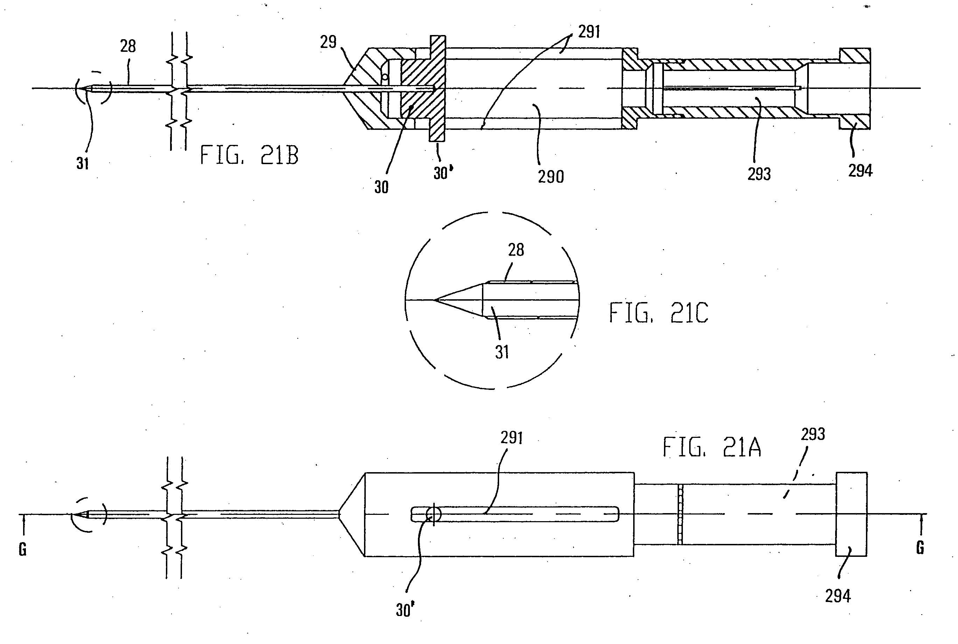

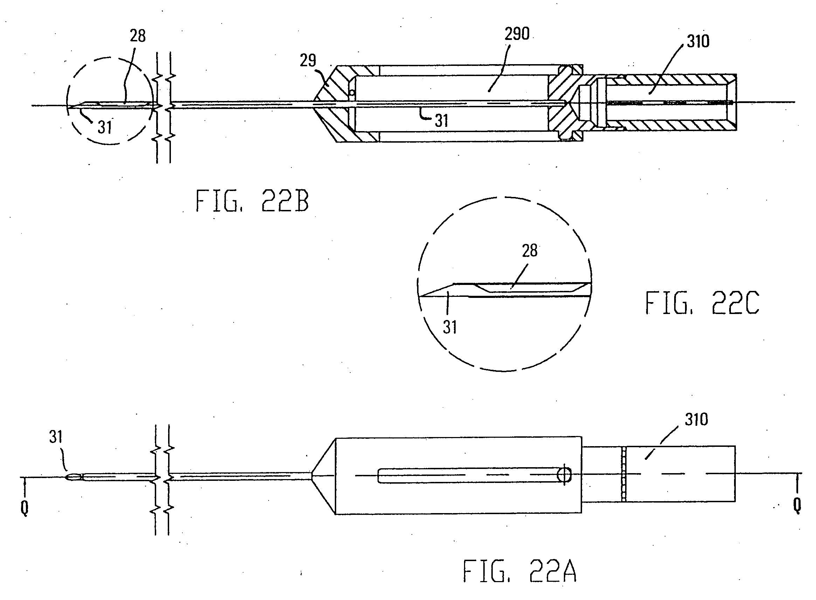

In particular, in comparison with the above examples of Figs 1-19B, the elements (3) (4) feature different structures which allow the use of needle-cannulae (28, 31) both of the type showed in Figs. 21A-21C and of the type showed in Figs 22A-22C.

As regards the body (3), it features a valve body (41) for the charging of a gas, with a gasket and a grub screw (43) for adjusting the precharge of internal spring (40) for the maintenance of valve (41).

Moreover, the body (3) features a distal web (300) to which the body (29) is associated. The latter extends backward, as described in Figs 21A and 21B and forms a cavity (290) in which the anchorage (30) of the stylet (31) is positioned - being said anchorage provided with appendages (30') which are placed in corresponding lateral eyelets (291) of said cavity (290), so as to slide through them - and it features a further extension and defining a seat (293) for the body (3) which is positioned in this seat during the assembly of the device. Said seat (293) features and end web (294).

In the examples of Figs 22A and 22B, the body (29) does not feature the above seat (293) but its cavity (290) hosts the support for the stylet (31), free to slide through this cavity and said support features a seat (310) for the body (3) in the back.

In the device illustrated in Fig 20B, the configuration of which allows cannula (28) to penetrate into the tissue and to draw the sample to be tested, when the bodies (3) and (2) are operated, the web (294) comes in contact with the surface (800) of the body (8) and places itself between the base of the body (2) and the appendages (400) of the body (4). Therefore, as the cannula moves forward, the stylet remains motionless. The forward motion of the cannula can be damped more or less intensely according to the position assumed by element (23). The tissue sample to be tested remains inside the cannula, afterwards the operator draws back the whole unit and causes the tear and the consequent removal of the sample.

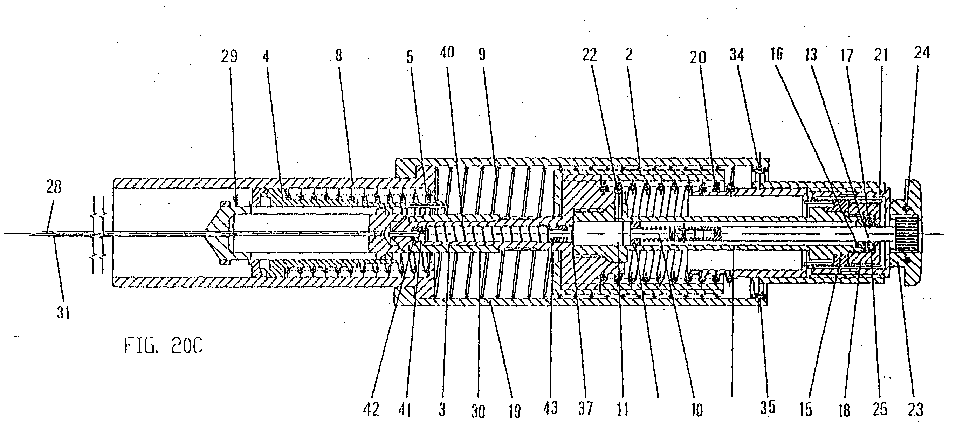

For the device illustrated in Fig. 20C, the configuration of which allows the forward motion both of the cannula and of the stylet, the operation is the same as that of the device described in Figs 1-19B where the appendages (400) can be unhooked and both the cannula and the stylet can move forward. The forward motion of the stylet, which can be damped more or less intensely, is allowed or not allowed according to the position of the element (23).

## Claims

1. Device for carrying out drawings, comprising means for drawing fragments or organic samples on diagnostic purposes, guide and support means for said drawing means, means for commanding a forward motion of said drawing means, **characterised in that** it comprises means for damping the forward motion of said drawing means.

2. Device according to claim 1, comprising a support group or unit (4) for a cannula (31) and a support group or unit (6) for a corresponding stylet (28) and means for operating the sequential forward motion of said support groups or units (6, 4) and, as a consequence of this, the sequential forward motion of said stylet (28) and of said cannula (31), **characterised in that** it comprises means for damping the forward motion of said stylet (28).

3. Device according to claim 1 or 2, **characterised in that** it comprises means for activating or deactivating said damping means.

4. Device according to claim 1 or 2 **characterised in that** it comprises means for operating the sequential automatic forward motion of said stylet (28) and of said cannula (31).

5. Device according to claim 1 or 2, **characterised in that** it comprises means for manually operating the forward motion of said stylet (28) and for operating the automatic forward motion of said cannula (31) at the end of the forward motion of the stylet (28).

6. Device according to claim 4 **characterised in that** said automatic operation means comprises buttons (212) placed on an external surface of the device.

7. Device according to one or more previous claims **characterised in that** said means for automatic operation of the forward motion of cannula (31) comprise a plurality of flexible and elastic appendages (400) presented on the back by a tubular body (4) functioning as support and guide for the forward motion of said cannula (31).

8. Device according to one or more previous claims **characterised in that** it comprises axial re-entry recharge means with reciprocal approach of two bodies (21, 8) which are at least partially placed inside a third tubular shaped body (19).

9. Device according to one or more previous claims **characterised in that** it comprises means for adjusting the action of the damping means for the forward motion of the drawing means.

10. Device according to claims 4 and 6 **characterised in that** it comprises elastic means (150) associated with said buttons (212) for controlling the forward motion of the stylet (28) by pressing the buttons.
